# EUROPÄISCHE PATENTANMELDUNG

(11) **EP 1 880 766 A1**
(43) Veröffentlichungstag der Anmeldung: **23.01.2008**
(21) Anmeldenummer: 07110140.6
(22) Anmeldetag: 13.06.2007
(51) Int. Cl.: B01L 3/00, B01J 19/00, C12Q 1/68

(54) **Auf porösem Material basierendes Analysesystem für hochparallele Einzelzelldetektion**

(30) Priorität: 21.07.2006 DE 102006033875
(71) Anmelder: SIEMENS AKTIENGESELLSCHAFT, 80333 München (DE)
(72) Erfinder: Ehben, Thomas, 91085 Weisendorf (DE); Zilch, Christian, Dr., 04275 Leipzig (DE)

(57) **Zusammenfassung**

Die vorliegende Erfindung bezieht sich auf Analysesysteme basierend auf porösem Material (1), z.B. Silizium, für hochparallele Einzelzelldetektion. Insbesondere bezieht sich die vorliegende Erfindung auf poröses Silizium (1) mit einer Vielzahl durchgehender Kanäle (2) und auf dessen Verwendung zumindest zur Zellseparation, zur Zelllyse und Aufreinigung von Zielmolekülen, zur Amplifikation von Nukleinsäuremolekülen oder zur Detektion von gesuchten Zielmolekülen. Die vorliegende Erfindung bezieht sich auch auf ein Analyseverfahren unter Verwendung von porösem Silizium (1). An den Innenwänden der Kanäle (2) sind monoklonale Antikörper (11) zur Zellseparation und immobilisierte Fängermoleküle (12) zur Zelllyse und Aufreinigung angebracht.

## Beschreibung

Die vorliegende Erfindung bezieht sich auf Analysesysteme basierend auf porösem Material, z.B. Silizium, Glas oder Kunststoff, für hochparallele Einzelzelldetektion. Insbesondere bezieht sich die vorliegende Erfindung auf poröses Material mit einer Vielzahl durchgehender Kanäle und auf dessen Verwendung zumindest zur Zellseparation, zur Zelllyse und Aufreinigung von Zielmolekülen, zur Amplifikation von Nukleinsäuremolekülen und/oder zur Detektion von gesuchten Zielmolekülen. Die vorliegende Erfindung bezieht sich auch auf ein Analyseverfahren unter Verwendung von porösem Material mit einer Vielzahl durchgehender Kanäle.

In der pharmazeutischen Forschung besteht ein wachsender Bedarf an Methoden und Technologien zur schnellen und akkuraten Analyse von Nukleinsäuren und Proteinen und anderen molekularen Zielstrukturen aus biologischen Proben. Die Entwicklung schneller, parallel durchführbarer Assays für Genom- und Proteomanalysen, Pharmakokinetik und Toxizitätsstudien ist ein wesentlicher Treiber pharmazeutischer und diagnostischer Produktentwicklungen. Darüber hinaus wächst der Bedarf nach Diagnostiksystemen zur hochparallelen genomischen und proteomischen Analyse von großen Zellpopulationen und einzelnen Zellen aus einem Gewebsverbund, beispielsweise einer Tumorbiopsie. Mit deren Hilfe können erhebliche Kosten eingespart werden und der Durchsatz durch den Einsatz parallel arbeitender und automatisierter Systeme maßgeblich erhöht werden. Sowohl in der klinischen Diagnostik und der Krankheitsforschung als auch in der Wirkstoffentwicklung werden zunehmend Mikroarrays angewandt, mit denen die Entschlüsselung krankheitsrelevanter Veränderungen in Zellen und Geweben möglich geworden ist.

Mithilfe von Biochips kann die Konzentration bzw. das Vorhandensein von Biomolekülen (z.B. DNA, Proteine) in biologischen Proben bestimmt werden. Bei Mikroarrays befinden sich spezifische Fängermoleküle, wie Oligonukleotide, Antikörper oder Haptene, an distinkten Orten (Spots) auf der Oberfläche geeigneter Träger (wie z.B. Glas, Plexiglas, Silizium). Der Vorteil von derzeit entwickelten Mikroarrays gegenüber konventionellen Analysemethoden (insbesondere homogener Assaysysteme) besteht vor allem darin, dass viele unterschiedliche biologische Fragestellungen in einem einzigen Experiment beantwortet werden können. Im Allgemeinen eignen sich konventionelle Mikroarrays zur Analyse vieler verschiedener biologischer Parameter in einer Probe. So können beispielsweise gleichzeitig die Expressionsprofile vieler Gene oder Proteine, eine große Anzahl von SNPs oder Genmutationen oder das Vorhandensein genetischer Sequenzen, exprimierter mRNA oder von Proteinen detektiert werden.

In aller Regel werden nach Extraktion von Proteinen oder Nukleinsäuren aus den Zellen einer Probe die nachzuweisenden Zielmoleküle an den spezifischen an einem Träger immobilisierten Fängermolekülen gebunden und mittels verschiedener Arten von Markern nachgewiesen. Dies erfordert eine Kopplung des jeweiligen Markers an das nachzuweisende Zielmolekül. Zur Detektion des jeweiligen Markers kommen sowohl optische als auch magnetische, elektrische oder gravimetrische Verfahren zum Einsatz. Die Empfindlichkeit dieser Verfahren ist sehr unterschiedlich. Generell lässt sich jedoch feststellen, dass hohe Konzentrationen von Zielmolekülen in einer Probe vorhanden sein müssen, um deren Detektion nach Bindung an Fängermoleküle zu ermöglichen. Bei den meisten auf Mikroarrays basierenden Verfahren ist deshalb eine große Menge an Ausgangs-Probenmaterial notwendig, um diese ausreichend hohe Konzentration von Zielmolekülen zu gewährleisten. Je nach Probe werden hierbei mehrere 100 µl oder µg Körperflüssigkeit (z.B. Blut) bzw. Gewebsmaterial (Biopsie oder Abstrich) benötigt, um die notwendige Anzahl von Zellen oder biologischen Partikeln, die die Zielmoleküle enthalten, zu gewinnen. In manchen Fällen ist jedoch nicht genügend Zellmaterial vorhanden oder der Anteil von nachzuweisenden (kranken) Zellen im gesamten Probenmaterial so gering, dass die Zielmoleküle vor dem Nachweis auf einem Mikroarray amplifiziert werden müssen.

In der Nukleinsäureanalytik kommen mehrere Amplifikationsverfahren, wie beispielsweise die Polymerase-Chain-Reaction (PCR; Polymerase-Kettenreaktion), die Ligase-Chain-Reaction (LCR) oder die Rolling-Cycle-Amplification zum Einsatz. Nachteilig ist jedoch, dass alle diese Verfahren in Bezug auf die Anzahl der gleichzeitig zu analysierenden Proben limitiert sind. Es ist zwar möglich, getrennte Amplifikationesreaktionen z.B. in den Kavitäten einer Mikrotiterplatte durchzuführen. In der nachfolgenden Hybridisierung auf einem Mikroarray kann jedoch nur eine einzige Probe gemessen werden. Zwar können viele verschiedene biologische Parameter jeder einzelnen Probe gleichzeitig bestimmt werden, jedoch erlauben die Designs von Mikroarrays derzeit keine differenzierte Messung biologischer Parameter aus einzelnen Zellen oder Zellen verschiedener gepoolter Proben. Dies ist darauf zurückzuführen, dass es sich bei den Trägern um plane Oberflächen, auf die die Fängermoleküle gespottet oder synthetisiert werden, handelt. Jede Probe (wie beispielsweise DNA oder RNA) benetzt die gesamte Oberfläche des Mikroarrays, so dass alle Spots mit der jeweiligen Probe in Kontakt geraten. Dieser Umstand bei Mikroarray-Experimenten schlägt sich in einem geringen Probendurchsatz nieder. In der Labormedizin, insbesondere beim Massenscreening, werden hingegen wenige biologische Parameter einer großen Anzahl von Proben bestimmt. Derzeit sind jedoch keine mikroarraybasierten Systeme im Einsatz, die es erlauben, multiple Parameter bei gleichzeitig hohem Probendurchsatz zu bestimmen.

Ein weiteres grundlegendes Problem stellt die Heterogenität einiger der verwendeten Probenmaterialien dar. Beispielsweise besteht eine Tumorbiopsie aus normalen Zellen und entarteten Zellen unterschiedlichen Malignitätsgrades. Bei bakteriologischen Proben handelt es sich in der Regel um Gemische verschiedener Erreger unterschiedlicher Pathogenität. Bei viralen Proben liegt hingegen latentes neben aktivem viralen Genmaterial in infizierten Zellen oder einer Körperflüssigkeit (Urin, Blutplasma, Lymphe) vor. Oftmals befinden sich die gesuchten Zellen oder biologischen Partikel in einer Minderzahl gegenüber anderen in der Probe vorkommenden "normalen" Zellen oder nichtpathogener Erreger. Die Schwierigkeit besteht vor allem darin, die mit einer Krankheit assoziierten Zellen oder Erreger vor dem Hintergrund "normaler" Zellen oder nicht-relevanter Bakterien oder Viren nachzuweisen ("Nadel-im-Heuhaufen-Problem"). Aufgrund der Heterogenität der in einer Probe vorhandenen Zelltypen erhält man selbst mit den spezifischen Amplifikationsverfahren wie beispielsweise PCR meist ein Ergebnis, das eine Mischkultur oder heterogene Probe repräsentiert. Dies ist insbesondere dann der Fall, wenn sich die Gensequenzen in den verschiedenen Zellpopulationen ähneln. Aus den genannten Gründen besteht ein Bedarf an kostengünstigen Verfahren, die es ermöglichen, multiple biologische Parameter aus heterogenen Zellpopulationen mit hohem Durchsatz zu bestimmen, und die eine akkurate statistische Analyse der genomischen bzw. proteomischen Informationen erlauben.

Um die die oben beschriebenen Probleme zu adressieren, werden derzeit die heterogenen Zellpopulationen oder biologischen Partikel mithilfe von Selektionstechniken wie Fluorescence-Activated-Cell-Sorting (FACS) oder funktionalisierten magnetischen Beads aufgetrennt. Falls es sich bei dem Probenmaterial um histologische Schnitte handelt, kommen Lasermikrodissektionsverfahren (Laser Microdissection, LMD) zu Einsatz, um spezielle Regionen innerhalb eines Gewebsschnitts herauszuarbeiten um die so gewonnenen Zellen dann weiteren Analyseverfahren wie Immunostaining oder molekularbiologischen Techniken zu unterziehen. In Quintessenz erzeugt man jedoch lediglich Mischungen selektierter Zelltypen oder biologischer Partikel. Eine Analyse auf Einzelzellbasis gestaltet sich außerordentlich schwierig. Dieser Umstand erweist sich insbesondere bei folgenden Applikationsfeldem als nachteilig:
a) Umfangreiche populationsgenetische Studien bedürfen der hochparallelisierten Abarbeitung biochemischer Prozesse, beginnend mit Zellsortierungs- und Aufschlussverfahren, Amplifikation der gesuchten genetischen Information, Detektion und Quantifizierung der Zielsequenzen. Eine Automatisierung der verschiedenen erforderlichen Schritte setzt eine aufwändige Laborinfrastruktur und Robotik voraus. Zurzeit werden die Schritte auf so genannten Mikrotiterplatten durchgeführt, die in unterschiedlichen Formaten kommerziell erhältlich sind. Die gängigen Formate reichen von 96, 384 bis 1536 well-plates, was die Anzahl der gleichzeitig prozessierbarer Proben limitiert. Die verschiedenen Prozesse bedürfen zudem eines ständigen Wechsels von Pufferflüssigkeiten in jedem einzelnen Töpfchen, was einer präzisen Robotersteuerung bedarf. Das Zeiterfordernis bei tausenden von Patientenproben liegt, je nach Plattenformat, Maschinendurchsatz und biologischer Fragestellung zwischen wenigen Tagen bis mehreren Wochen. Dies bedeutet abgesehen vom enormen Zeitaufwand hohe Material- und Personalkosten.
b) Bei der Kartierung von Tumoren werden histologische Gewebsschnitte angefertigt, die mit Farbstoffen behandelt werden, die eine Unterscheidung von verschiedenen Zell- und Gewebstypen ermöglichen. Neuerdings kommen monoklonale Antikörper zum Einsatz, um spezielle intra- und extrazelluläre Strukturen, die Tumorzellen auszeichnen, zu identifizieren. Auf diese Weise ist es möglich, den Differenzierungsgrad und das Metastasierungspotential von entarteten Zellen und die Grenze zu gesunden Zellen innerhalb des befallenen Organs zu bestimmen. In letzter Zeit gewinnen genetische Analyseverfahren zunehmend an Bedeutung, da diese zusätzliche Informationen über die Tumorzellen und deren Empfindlichkeit gegenüber Chemotherapeutika liefern können. Dazu werden histologisch auffällige Regionen in Schnitten mit Hilfe von Laser-Dissektionsverfahren herausgelöst und einer genetischen Analyse unterzogen. Hierbei werden im Allgemeinen Genexpressionsprofile erstellt, die eine Aussage über den Stoffwechsel von Tumorzellen erlauben, oder Mutationsprofile, die dem behandelnden Arzt Hinweise auf die betroffenen Gene geben und so eine Kategorisierung des Tumors ermöglichen. Das Problem bei den heute verwendeten Verfahren ist die Tatsache, dass sich eine Verarbeitung histologischer Schnitte sehr aufwändig gestaltet und hoch qualifiziertes Personal erfordert, um zu einem aussagekräftigen Ergebnis zu gelangen. Oftmals werden bei histologischen Schnitten die relevanten Regionen in Tumoren nicht erfolgreich identifiziert, was zu Falschaussagen über den Differenzierungs- und Metastasierungsgrad oder dessen Empfindlichkeit gegenüber Chemotherapeutika führen kann.
c) Die Bestimmung der Anzahl der Partikel und infizierten Wirtszellen ist bei der Diagnose und des Monitoring viraler Erkrankungen von herausragender Bedeutung. Insbesondere im Kontext einer fortschreitenden Pathogenese spielen die Anzahl der latent infizierten Zellen und die Dynamik der Virenproduktion eine wichtige Rolle. Es werden deshalb eine Reihe viraler Assays entwickelt, die eine Aussage über die Anzahl der viralen Kopien (viral load) in einer definierten Menge an Körperflüssigkeit ermöglichen. Diese Information gibt wichtige Hinweise über den Verlauf einer viralen Erkrankung oder einen Erfolg einer antiviralen Therapie. Ein Problem stellt jedoch die Unterscheidung zwischen latent infizierten, aber inaktiven Wirtszellen und denen, die aktiv virale Partikel produzieren, dar. Die zur Zeit verwendeten Methoden zur Viral-Load-Bestimmung ermöglichen zwar die ungefähre Bestimmung viraler Kopien (RNA oder DNA) in einer definierten Menge an Körperflüssigkeit, jedoch ist es bisher nicht möglich, die Anzahl der tatsächlich infizierten Zellen in einer Zellpopulation zu bestimmen. Ferner gestaltet es sich außerordentlich schwierig, von der Anzahl der amplifizierten viralen Kopien auf die Anzahl infizierter Zellen zu schließen. Dies würde eine Analyse jeder einzelnen Zelle einer Population erfordern. Eine Einzelzellbestimmung ist mit heute verwendeten empfindlichen Amplifikationsverfahren zwar möglich, eine sinnvolle Statistik erweist sich aber aufgrund der verschwindend geringen Zahl infizierter Zellen in einer Population nichtinfizierter Zellen oftmals als nicht praktikabel. Eine Reihe von derzeit in der Entwicklung befindlichen Selektionsmethoden bedient sich neuartiger mikrofluidischer Konzepte.

Beispielsweise hat das Fraunhofer-Institut für Biomedizinische Technik (IBMT) so genannte Mikrokapillar-Biochips entwickelt, die es erlauben, Einzelzellen und Gewebeproben für funktionelle Proteomik und Biomonitoring zu analysieren. Die Systeme ermöglichen ein automatisiertes Screening von toxikologischen Substanzen an kleinsten Zell- und Gewebemodellen unter Einsatz der Impedanzspektroskopie. Anwendungsmöglichkeiten ergeben sich bei der pharmazeutischen Wirkstoffsuche, bei der Qualitätskontrolle in der Lebensmitteltechnologie oder in der Umwelttechnologie. Der Durchsatz ist jedoch auf wenige hundert Proben pro Tag beschränkt.

Das IZKF Leipzig hat eine neue Plattformtechnologie entwickelt, mit der es möglich wird, ein miniaturisiertes Labor zur Zellforschung auf einem Mikrochip einzurichten. Dort können, wie auf einem Rangierbahnhof, einzelne Zellen in einem elektrischen Käfig schonend gehalten, sortiert, charakterisiert und behandelt werden. Eine ähnliche Technologie wurde am CEA in Grenoble im Rahmen des MeDICS- Projekts entwickelt. Mit den miniaturisierten Zellsortierem können Zellen ohne mechanischen Kontakt mithilfe von dielektrophoretischen Feldern manipuliert werden. Mit beiden Technologien ist eine nachfolgende Genom- und Proteomanalyse der Einzelzellen zwar prinzipiell möglich, jedoch derzeit noch im Experimentalstadium und noch nicht in diagnostische Systeme integriert. Der Probendurchsatz ist aufgrund der seriellen Sortierung ebenfalls eingeschränkt.

Sehr sensitive Amplifikationsverfahren erlauben zwar mittlerweile eine Analyse der sortierten Zellen auf Einzelzellbasis, jedoch ist dafür jeweils ein separater Assay notwendig. Einzelzellsysteme zur Hochdurchsatzanalyse werden von verschiedenen Unternehmen, darunter Evotec OAI, Cybio, Tecan, Beckman Coulter, Guava Technologies etc. angeboten. Sämtliche Verfahren sind jedoch auf einen Durchsatz von wenigen Tausend Proben oder Zellen pro Durchlauf beschränkt, d.h. es ist derzeit nicht möglich, parallel und in kurzer Zeit mehrere Millionen Zellen einer Einzelzellanalyse zu unterziehen. Lediglich eine geringe Anzahl von Einzelzellanalysen verläuft mit den derzeit in der Anwendung befindlichen Methoden erfolgreich.

Histologische Schnitte von Tumormaterial, komplexe Populationsstudien oder die Erregerdifferenzierung bei Mischkulturen beinhalten jedoch meist mehrere Millionen verschiedener Zellen, um zu einer sinnvollen statistischen Aussage zu gelangen. Die o.g. Verfahren sind für diese Fragestellungen zu aufwändig, langwierig, teuer oder technisch nicht praktikabel, um eine molekulare Analyse auf Einzelzellbasis für eine hohe Anzahl verschiedener Zelltypen gleichzeitig durchzuführen.

Die US 5,843,767 offenbart einen Flow-Through-Chip mit einer Vielzahl von diskreten Kanälen, die sich von einer ersten Oberfläche bis zu einer gegenüberliegenden zweiten Oberfläche erstrecken. Die Kanäle weisen einen Durchmesser von etwa 0,033 µm bis etwa 10 µm auf. Ein erstes Bindereagenz zum Binden eines Zielmoleküls ist an den Wänden der Kanäle immobilisiert. Das Bindereagenz ist geeignet zum Ausführen einer analytischen Aufgabe zur Profilerstellung von Zellpopulationen. Zur Detektion wird eine PCR Reaktion vorgeschlagen, wobei dem Flow-Through-Chip ein hierfür notwendiges Heizelement fehlt.

Aus der Patentschrift DE 101 42 691 ist ein Analyseverfahren mit einem makroporösem Substrat bekannt, das gegenüberliegend eine erste und zweite Oberfläche aufweist, wobei über einen Oberflächenbereich eine Vielzahl von diskreten Poren miteinem Durchmesser von 500 nm bis 100 µm angeordnet ist, welche sich durch das Substrat von der ersten zur zweiten Oberfläche erstrecken. Ein Analyt wird ortsspezifisch von mindestens einem Fängermolekül an den Innenwandoberflächen pro Pore immobilisiert. Dann wird in Abhängigkeit einer Bindungsreaktion zwischen Fängermolekül und dem Analyten eine sich ändernde Licht-Transmissionseigenschaft der Pore gemessen. Der Analyt ist beispielsweise eine einzelne Zelle. Dazu wird Licht in die Pore eingekoppelt und von einem CCD-Array an der gegenüberliegenden Seite detektiert. Diese Änderung der Licht-Transmissionseigenschaft ist bei Verzicht auf einen Amplifizierungsschritt des Analyten naturgemäß nur mit hohem Instrumentellem Aufwand bei mäßigem Signal-Rausch-Verhältnis zu detektieren.

Ein weiteres System zur Verwendung eines Flow-Through-Chips mit einer Kapillarenkassette ist aus der DE 200 22 783 U1 bekannt. Die Kapillarkassette weist ein Substrat und eine Mehrzahl von Kapillaren auf, welche durch das Substrat reichen und je ein offenes Ende auf entgegengesetzten Seiten des Substrats aufweisen. Zur Amplifizierung von Nukleinsäure mittels PCR Reaktion werden die offenen Enden mit einer Membran verschlossen. Zur Durchführung der Reaktion wird die Kassette in einen Thermozykler eingebracht. Mittels eines Luftstroms, der von einem Heizelement im Thermzykler erwärmt wird, ist eine PCR Reaktion durchführbar. Besonders nachteilig an diesem System ist, dass die Reaktionsprodukte zur Detektion aus den Kapillaren ausgeteilt werden müssen. Weiterhin erzeugt ein an die Kapillare gebundener Analyt ein signifikant erniedrigtes Signal bei der Detektion als ein ungebundener Analyt bei identischen Bedingungen.

Es ist die Aufgabe der vorliegenden Erfindung, Mittel vorzusehen, mit denen die vorstehend beschriebenen Analysen preiswerter und einfacher und mit höherem Durchsatz durchzuführen sind.

Diese Aufgabe wird durch einen Flow-Through-Chip aus einem porösen Material gemäß Anspruch 1 gelöst. Die Erfindung ist ferner auf eine Verwendung eines Fow-Through-Chips aus porösem Material sowie auf das Analyseverfahren mit den Merkmalen von Anspruch 7 gerichtet.

Vorteilhafte Weiterbildungen sind in den Unteransprüchen definiert.

Erfindungsgemäß wird vorzugsweise die bereits etablierte Flow-Through-Chip Lösung basierend auf porösem Silizium, die bisher für Genexpressionsstudien verwendet wird, so modifiziert, dass völlig neue Anwendungsbereiche, wie die hochparallelisierte Einzelzellanalyse, erschlossen werden können. Das poröse Material kommt bei den neuen Verfahren vorzugsweise als Nanotiterplatte oder -Chip zum Einsatz, in der jeder einzelne, durchgehende Kanal zur Zellseparation, als Aufschlusskanal, Amplifikationskanal und Detektionskanal verwendet werden kann. Abgesehen von einer hochparallelisierten Abarbeitung vieler Proben ist eine Analyse einzelner Zellen auf genomischer und potenziell auch auf proteomischer Basis möglich.

Das poröse Material kann jedes Material sein, welches eine Vielzahl von durchgehenden Mikrokanälen mit einem Durchmesser in der Größenordnung im Bereich von 0,1 bis 100 µm aufweisen kann. Die Mikrokanäle sind vorzugsweise im wesentlichen parallel zueinander. Somit kann das Material sowohl poröses Silizium sein, wie weiter unten im Ausführungsbeispiel beschrieben, als auch poröses Glas oder poröser Kunststoff. Geeignetes poröses Glas wird z. B. dadurch hergestellt, dass ein Bündel aus Glasfasern wiederholt erwärmt, in die Länge gezogen und komprimiert wird. Alternativ ist es auch möglich, einen porösen Siliziumchip zu Glas zu oxidieren, z.B. durch langes Erhitzen in geeigneter Atmosphäre.

Als poröser Kunststoff ist z.B. Epoxydharz geeignet, da dieser gute Bindungseigenschaften zu biologischen Molekülen aufweist. Die poröses Struktur kann durch verschiedene Verfahren hergestellt werden, z. B. durch additive Verfahren aus dem "Rapid Manufacturing" Bereich (Mikrostereolithographie), durch Mikroformtechnik, bei welchem ein geeignetes Gusswerkzeug mit Methoden der Mikrosystemtechnik hergestellt wird, oder durch abrasive Bearbeitungsverfahren wie SU-8-.

Mit Systemen wie z.B. dem von Infineon und Metrigenix entwickelten Flow-Through-System war es bisher nicht möglich, viele Proben gleichzeitig zu untersuchen. Zusätzlich musste die Probenaufbereitung noch separat durchgeführt werden. Erst nach Isolierung der Nukleinsäuren und deren Markierung werden alle weiteren Schritte der Hybridisierung und nachfolgenden Detektion mithilfe der 3-D- bzw. 4-D-Fluidiksysteme gesteuert. Bei der Erfindung ist es jedoch in vorteilhafter Weise möglich, sowohl die Zellbindung als auch die Lyse, Nukleinsäureisolation, Amplifikation und den Nachweis innerhalb jedes einzelnen Kanals durchzuführen. Dazu werden in jedem einzelnen Kanal an der Wandung Antikörper, die gegen eine gesuchte Zelle oder das Zielprotein gerichtet sind, befestigt. Für genomische Anwendungen in der Nukleinsäurediagnostik werden an die Kanalinnenwände einzelsträngige Oligonukleotide fixiert, die als Fänger Gensequenzen in jeder einzelnen Zelle zu binden vermögen. Zur Steuerung der Mikrofluidik kann vorzugsweise ein modifiziertes, von Infineon entwickeltes 3-D-Flow-Through-System zum Einsatz kommen.

Mit Bezug auf die beiliegenden Zeichnungen werden nunmehr bevorzugte Ausführungsbeispiele der Erfindung beschrieben.

In den Zeichnungen zeigen
- Fig. 1: ein Ausführungsbeispiel von porösem Material in der Form eines Flow-Through-Chips, der gleichzeitig die Form einer Nanotiterplatte aufweist;
- Fig. 2: eine Anordnung des Flow-Through-Chips gemäß der Fig. 1 in einem Analysesystem;
- Fig. 3: einen histologischen Schnitt, der auf dem Flow-Through-Chip gemäß der Fig. 1 angeordnet ist;
- Fig. 4: ein Beispiel einer virologischen Applikation unter Verwendung des Flow-Through-Chips gemäß der Fig. 1;
- Fig. 5: eine Querschnittsansicht des Flow-Through-Chips gemäß der Fig. 1, wobei statistisch eine einzelne Zelle an einem Antikörper in dem Kanal gebunden wird;
- Fig. 6: eine vergrößerte Querschnittsansicht der Fig. 5, wobei eine DNA der Zelle freigesetzt wird; und
- Fig. 7: eine vergrößerte Querschnittsansicht der Fig. 5, wobei eine PCR und eine Detektion von gesuchten Molekülen durchgeführt werden.

Im Folgenden werden die Ausführungsbeispiele der vorliegenden Erfindung unter Bezugnahme auf die Zeichnungen beschrieben.

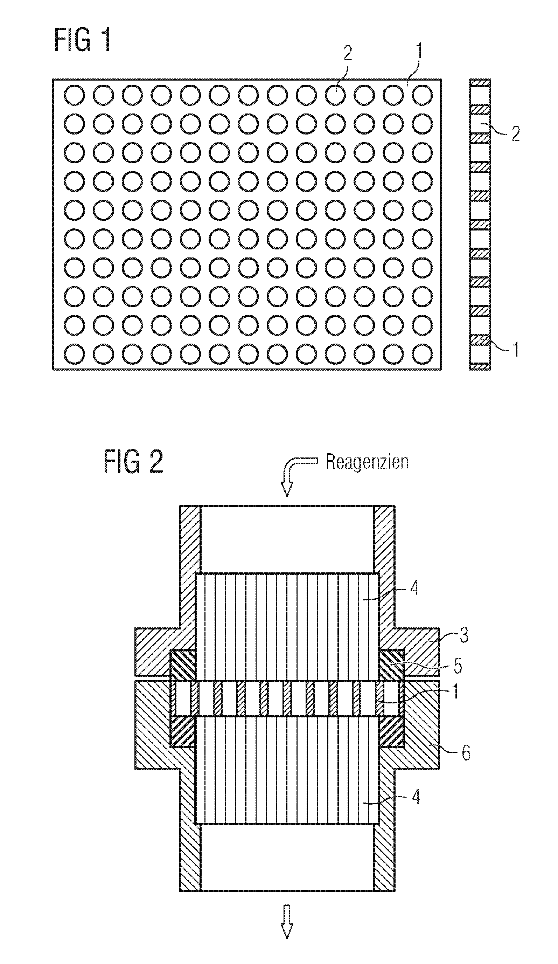

Die Fig. 1 zeigt ein Ausführungsbeispiel von porösem Material in der Form eines Flow-Through-Chips 1 aus Silizium, der gleichzeitig die Form einer Nanotiterplatte aufweist, wobei jedoch die Kanäle 2 den Flow-Through-Chip 1 vollständig durchdringen.

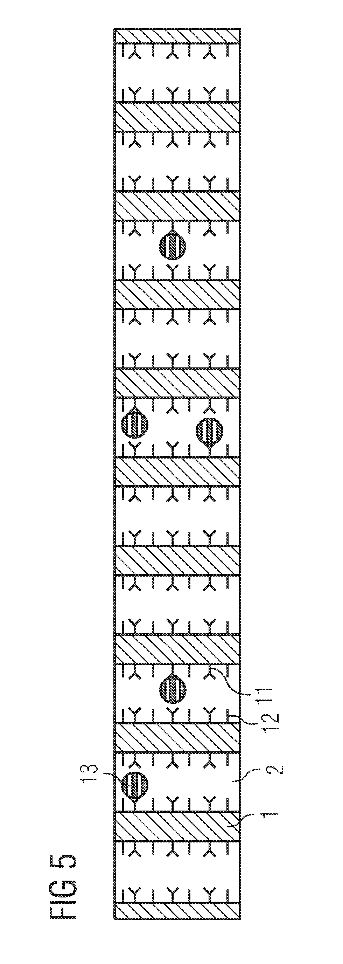

Das bei diesem Flow-Through-Chip 1 eingesetzte poröse Silizium besteht aus einem einige hundert Mikrometer dicken Einkristallplättchen, in das pro Quadratzentimeter Fläche einige hunderttausend gleich beschaffene Kanäle 2 eingeätzt sind. Die Kanäle 2 durchziehen das Plättchen des Flow-Through-Chips 1 vollständig, d.h. sie reichen von der Ober- bis zur Unterseite. Der Durchmesser der Kanäle 2 kann durch Wahl der Ätzbedingungen ca. zwischen 0,1 und 100 µm gewählt werden. Die Bedingungen müssen so gewählt werden, dass eine einzelne eukaryontische oder prokaryontische Zelle 13 darin Platz findet, wie dies z.B. in der Fig. 5 angedeutet ist. Es müssen zwei weitere wichtige Voraussetzungen erfüllt werden: 1) Die Kanäle 2 müssen von der Ober- und Unterseite des Flow-Through-Chips 1 variabel, jedoch nicht unabhängig voneinander verschließbar sein und 2) eine Durchleitung von Flüssigreagenzien ermöglichen.

Die Fig. 2 zeigt eine derartige Anordnung des Flow-Through-Chips 1 gemäß der Fig. 1 in einem Analysesystem.

Die Verschließbarkeit wird vorzugsweise durch einen Dichtungsschwamm 4 und optional durch eine obere und eine untere verschiebbare Deckelplatte 3, 6 aus Polymermaterial oder Membranen realisiert. Der Dichtungsschwamm 4 ist unter Druck dicht, um so eine Längsströmung zu verhindern, und kann z.B. durch die Deckelplatten 3, 6 bis zur Dichtheit komprimiert werden. Ferner klemmen die Deckelplatten 3, 6 den Flow-Through-Chip 1 mit einer dazwischen angeordneten Dichtung 5 ein. Optional können an der Dichtung 5 elektrische Kontakte und Leiter angebracht sein, damit die Dichtung 5 bei einer angelegten elektrischen Spannung als ein Heizelement zum Erwärmen des Flow-Through-Chips 1 dient. Stromabwärts und stromaufwärts von dem Flow-Through-Chip 1 ist ein Dichtungsschwamm 4 angeordnet.

Die Durchleitung verschiedener Reagenzien wird mittels einer Durchflusskammer in den Deckelplatten 3, 6 gewährleistet, in die die Reagenzien aus den jeweiligen Vorratsbehältern (nicht gezeigt) hineingepumpt werden.

In jedem der Kanäle 2 finden räumlich voneinander getrennte biologische Reaktionen statt. Je nach Probenmaterial (Zellsuspension oder Gewebsschnitt) muss das Aufarbeitungsverfahren so gewählt werden, dass eine Vereinzelung der Zellen und eine Verteilung auf die verschiedenen Kanäle 2 des porösen Siliziums 1 erfolgt. Ein Ausführungsbeispiel eines Analyseverfahrens für eine Einzelzellanalyse wird im Folgenden unter Bezugnahme auf die folgenden Figuren beschrieben.

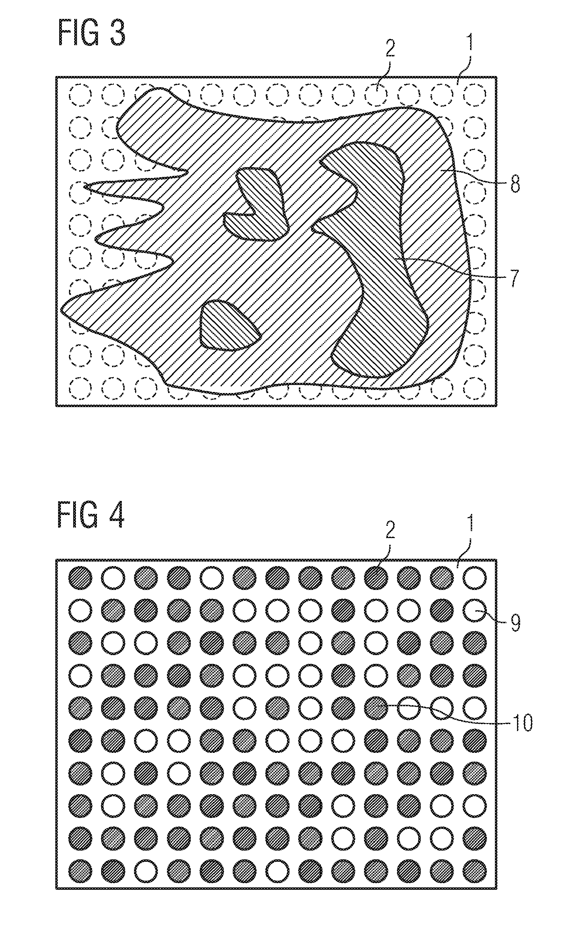

Die Fig. 3 zeigt einen histologischen Schnitt 8, der auf dem Flow-Through-Chip 1 angeordnet wird. In dem histologischen Schnitt befinden sich Tumorzellen 7, die es zu identifizieren gilt.

Die Fig. 4 zeigt alternativ ein Beispiel einer virologischen Applikation unter Verwendung des Flow-Through-Chips 1. Die leer dargestellten Kanäle 2 enthalten gesunde Zellen 9, und die voll dargestellten Kanäle 2 enthalten latent infizierte Zellen 10.

Im nächsten Schritt wird eine Zellseparation durchgeführt. Die Fig. 5 zeigt eine Querschnittsansicht des Flow-Through-Chips 1, wobei eine Zelle 13 an einem Antikörper 11 in dem Kanal 2 gebunden wird.

Dabei wird zunächst z.B. der histologische Schnitt 8 auf der Oberfläche des porösen Siliziumchips 1 chemisch fixiert. Nach der Inkubation in einem geeigneten Lysereagenz, das den Gewebsverbund auflöst (z.B. Trypsinierung oder Verwendung einer anderen geeigneten Protease) werden die Zellen 13 vereinzelt und vorzugsweise mithilfe eines Stempels, der auf das Gewebe wirkt, mechanisch in die Kanäle 2 gedrückt.

Verwendet man hingegen Zellsuspensionen, so sorgen die an der Innenwand der Kanäle 2 gekoppelten monoklonalen Antikörper 11 dafür, dass nach dem Einpumpen der Suspension durch das poröse Silizium 1 statistisch jeweils eine einzige Zelle 13 pro Kanal 2 gebunden wird. Hierzu wird ein Teil jedes einzelnen Kanals 2 mit aktiven chemischen Gruppen (Tosyl, Amino, Epoxy, Thiol etc.) funktionalisiert und mit spezifischen monoklonalen Antikörpern 11 bespottet, so dass die Innenwände der Kanäle 2 mit Antikörpern 11 ausgekleidet werden. Diese dienen der Bindung spezifischer Zielzellen, beispielsweise Lymphozyten, wie CD4- oder CD8-T-Helferzellen oder cytotoxischer Zellen. Auf diese Weise ist es möglich, unterschiedliche Zellpopulationen aus einer Mischung voneinander zu trennen und weiter zu verarbeiten.

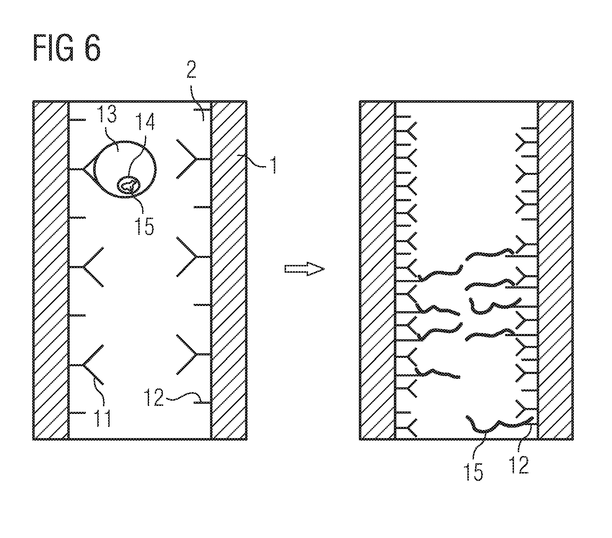

Im nächsten Schritt kann eine Zelllyse und Aufreinigung der Zielmoleküle durchgeführt werden, wie es in der Fig. 6 dargestellt ist. Die Fig. 6 zeigt eine vergrößerte Querschnittsansicht der Fig. 5, wobei eine DNA 15 der Zelle 13 freigesetzt wird. Mit dem Bezugszeichen 14 ist ein Zellkern der Zelle 13 bezeichnet.

Die in den Kanälen 2 befindlichen Zellen 13 werden nun chemisch, biologisch oder thermisch innerhalb eines jeden Kanals 2 lysiert. Die freigesetzten Proteine oder Nukleinsäuren werden durch an der Innenwand der Kanäle 2 immobilisierte Fängermoleküle 12, wie Antikörper oder Oligonukleotide, gebunden. Im Fall eines genomischen Assays werden beispielsweise Lysereagenzien durch die Kanäle 2 geleitet, die eine Freisetzung der RNA und DNA 15 bewirken. Die genetische Information kann mithilfe von an den Kanälen 2 gebundenen Silanen oder spezifischen Oligonukleotiden 12 gebunden werden. Im Fall von proteomischen Assays werden die Zielproteine beispielsweise durch immobilisierte spezifische Antikörper 12 an der Innenwand der Kanäle 2 gebunden. Anschließend erfolgt der direkte oder indirekte Nachweis durch die Detektion, oder, falls erforderlich, eine zwischengeschaltete Amplifikation. Die Amplifikation kann insbesondere bei einer Analyse von Nukleinsäuremolekülen 15 erforderlich sein, es sind jedoch auch Nukleinsäure-Assays denkbar, bei denen so viel biologisches Material erzeugt wird, dass auf eine Amplifikation verzichtet werden kann.

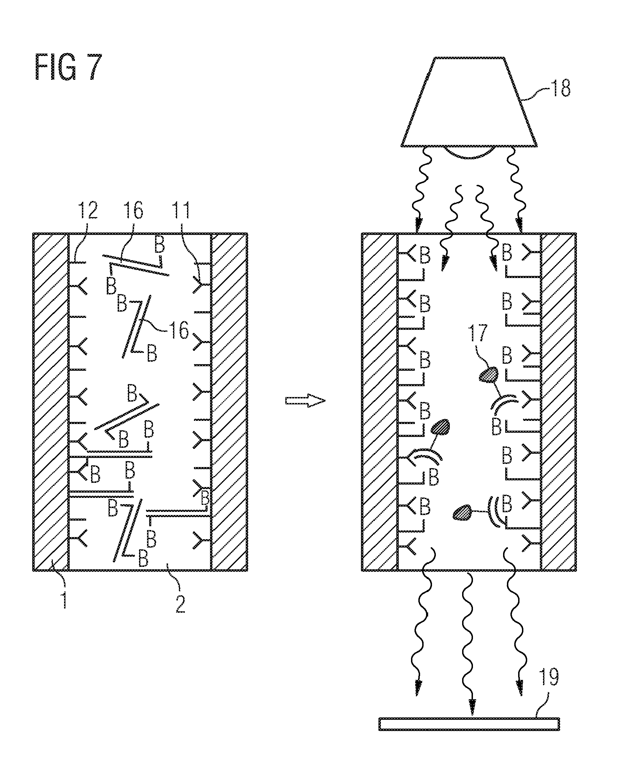

Die Amplifikation von Nukleinsäuremolekülen ist im linken Teil der Fig. 7 dargestellt. Die Fig. 7 zeigt eine vergrößerte Querschnittsansicht der Fig. 5, wobei eine PCR (Polymerase-Kettenreaktion) zur Amplifikation von gesuchten Molekülen 15 durchgeführt wird.

Aufgrund der hervorragenden Wärmeleitungseigenschaften des porösen Siliziums 1 ist man in der Lage, eine temperaturgesteuerte PCR in den einzelnen Kanälen 2 durchzuführen. Dies geschieht zum Beispiel durch thermische Ankopplung eines Peltierelements (nicht gezeigt) an den porösen Siliziumchip 1 und/oder durch elektrische Aufheizung aufgrund des Widerstands des Materials (z.B. Halbleitermaterials). Optional kann die Aufheizung auch durch Aufheizen der Dichtung 5 erfolgen, wenn diese, wie auf mit Bezug auf Fig. 1 beschrieben, leitfähig ist und mit elektrischen Kontakten versehen ist. Zuvor werden die notwendigen PCR-Reagenzien in die Kammern gepumpt. Zuvor an den Innenwänden der Kanäle 2 befestigte und vorzugsweise einzelsträngige Oligonukleotide, die z.B. mit Biotin 16 markiert werden, können zu einer Festphasen-PCR oder Primer-Extension verwendet werden. Es muss gewährleistet werden, dass die Reagenzien während der Amplifikation der Zielmoleküle nicht durch Diffusion entweichen. Dies kann beispielsweise durch die beidseitige Abdichtung oder Verdeckelung der Kanäle 2 mithilfe von Membranen oder Deckelplatten 3, 6 nach dem Einpumpen der erforderlichen Reagenzien erreicht werden, wie dies in der Fig. 2 dargestellt ist.

Alternativ, aber mit höherem manuellem Aufwand verbunden, kann der poröse Siliziumchip 1 in einer Art Sandwich zwischen zwei mit dünnen Dichtungen aus wärmeleitfähigem Material versehenen Peltierelemente eingespannt werden (nicht gezeigt). Innerhalb der Kanäle 2 des porösen Siliziumchips 1 kann auf diese Weise eine PCR, ähnlich wie in einer Mikrotiterplatte, jedoch mit sehr viel weniger Probenmaterial (in der Regel aus einer einzigen Zelle 13 pro Kanal 2) und wesentlich höher parallelisiert ablaufen (mehrere hunderttausend getrennter Reaktionen pro Quadratzentimeter Silizium).

Nach der Zelllyse oder nach der gegebenenfalls durchgeführten Amplifikation erfolgt die Detektion der gesuchten Zielmoleküle, wie sie im rechten Teil der Fig. 7 dargestellt ist. Der Nachweis gesuchter Zielmoleküle kann wie auch bei den bisher realisierten Applikationen mithilfe von optischen Nachweisverfahren (Fluoreszenz oder Biolumineszenz) erfolgen. Bei der Anordnung im rechten Teil der Fig. 7 wird der Kanal 2 durch eine Lichtquelle 18 beleuchtet. Am anderen Ende des Kanals 2 ist ein Detektor 19, vorzugsweise ein CCD-Chip oder eine CMOS-Kamera, angeordnet.

An den Innenwänden der Kanäle 2 des porösen Siliziumchips 1 befinden sich vorzugsweise immobilisierte spezifische Fängermoleküle 17, die mit den gesuchten Zielmolekülen in Interaktion treten können. Bei proteomischen Applikationen eignen sich hierfür Antikörper oder Haptene. Im Fall von genomischen Anwendungen sind dies Oligonukleotide, die mit komplementären Sequenzen der Zielmoleküle hybridisieren. Beim Nachweis spezifischer Mutationen in einem Sequenzabschnitt (SNPs) ist je nach Sequenzbehandlung eine Stringenz erforderlich, die durch die Pufferbedingungen und die Temperatur eingestellt werden kann.

Die ungebundenen markierten Moleküle müssen im Anschluss an den Anbindungs- oder Hybridisierungsvorgang aus den Kanälen durch Hindurchpumpen von Puffer herausgewaschen werden. Anschließend werden die verbleibenden über die Zielmoleküle mit den Fängermolekülen 17 gebundenen Marker mittels optischer Verfahren quantifiziert. Dies geschieht beispielsweise durch Einlegen des Siliziumchips 1 in einen optischen Scanner. Analog zu den bereits von Infineon und Metrigenix entwickelten Biolumineszenzverfahren kann auch die enzymatische Verstärkung bei lumineszierenden Agenzien zum Einsatz kommen. Alternativ können hierbei auch konventionelle magnetische, elektrische oder andere Verfahren bei der Detektion der Zielmoleküle verwendet werden. Weiterhin ist eine Schmelzkurve realisierbar, d.h. es kann ein definierter Temperaturbereich abgefahren werden, innerhalb dessen der Grad der Hybridisierung dynamisch bestimmt werden kann.

Die vorliegende Erfindung ist bei folgenden Anwendungen vorteilhaft:

Es können Millionen verschiedener einzelner Zellen aus unterschiedlichen Proben, wie Tumorgewebe, Mischpopulationen aus Blutproben oder anderen Körperflüssigkeiten hochparallel auf genomischer oder proteomischer Ebene charakterisiert und analysiert werden. Das System kann zur vollintegrierten Analyse von DNA und Proteinen zum Einsatz kommen. Daher eignet sich das Verfahren insbesondere für folgende Applikationen:
- Genexpressionsprofile auf Einzelzellbasis
- Erkennung von mutierten oder abartigen Zellen (Krebszellen) in einem komplexen Zellgemisch
- Analyse von heterogenen Zellgemischen
- Analyse von gepoolten Proben (Populationsgenetik)
- High-Throughput-Drug-Screening zur Identifizierung geeigneter Substanzkandidaten mittels konventioneller Fluoreszenz- oder Lumineszenz-Auslesung oder konfokaler Einzelzell- oder Moleküldetektion
- Verwendung des Flow-Through-Chips für massenspektrometrische Untersuchungen. Hierzu findet die Primer-Extension an der festen Phase innerhalb der einzelnen Kanäle statt

Gegenüber konventionellen Analysesystemen zeichnet sich die vorliegende Erfindung durch folgende vorteilhafte Eigenschaften aus:
- Eine Integration vieler verschiedener Prozesse von der Zellseparation, Nukleinsäurepreparation, Amplifikation bis zur molekularen Detektion wird möglich werden. Derzeit finden lediglich die Hybridisierung und die Lumineszenzreaktion in den Kanälen des Chips statt.
- Es wird möglich einzelne Zellen in jedem Kanal separat zu untersuchen. Auf diese Weise kann eine Zuordnung von Mutationen und Expressionsprofilen von Genen zu der jeweiligen Einzelzelle realisiert werden. Dazu könnten z.B. spezifische Antikörper in den Kanälen platziert werden, die eine Zellanhaftung ermöglichen.
- Die neuartigen Biochips aus porösem Silizium lassen sich in der Massenspektrometrie einsetzen, indem die benötigten Matrizes in die einzelnen Kammern eingebracht werden. Auf diese Weise kann eine wesentlich höhere Anzahl von Proben / genetische Parameter analysiert werden, als dies bei derzeitigen MALDI-Verfahren ("Matrix assisted laser dissociation and ionisation") der Fall ist.
- Der Workflow wird gegenüber laborgängigen Systemen maßgeblich vereinfacht, da keine aufwändige Robotik vonnöten ist. Bei der Verwendung der Technologie zur Untersuchung histologischer Schnitte ist keine aufwändige Laserdisektion mehr erforderlich. Histologische Färbungen und die nachfolgende genetische Untersuchung aller Regionen des gesamten histologischen Schnitts sind auf einem Chip (mit entsprechenden Ausmaßen) möglich.
- Bei der Anwendung für Viral-Load-Assays wird eine Aussage über die Anzahl latent infizierter und aktiv virusproduzierender Wirtszellen möglich. Man ist somit in der Lage, zu bestimmen, ob eine gewisse Anzahl viraler Kopien auf wenige aktive Zellen oder viele latent infizierte Zellen zurückzuführen ist.
- Die Testdauer wird aufgrund der enormen Parallelisierbarkeit signifikant reduziert. Die Parallelisierung von genomischen Assays, wie sie derzeit mit immer dichteren Mikrotiterplatten vorangetrieben wird, wird durch den Einsatz des porösen Siliziums noch einmal massiv vervielfacht. Es wird theoretisch möglich, mehrere Millionen separater Reaktionen auf wenigen Quadratzentimetern Chipoberfläche zu realisieren. Damit werden bessere statistische Auswertungen von Massenscreenings ermöglicht.
- Die Kosten für Laborrobotik und insbesondere für die Reagenzien werden signifikant reduziert, da einerseits die Geräte kleiner werden und andererseits die erforderlichen Reagenzienvolumina signifikant reduziert werden können.

Das hierbei beschriebene erfindungsgemäße Verfahren weist viele Schritte zur Zellseparation, zur Zelllyse und Aufreinigung von Zielmolekülen, zur Amplifikation von Nukleinsäuremolekülen sowie zur Detektion von gesuchten Zielmolekülen auf. Jedoch ist die vorliegende Erfindung nicht auf die hier beschriebene Kombination der einzelnen Schritte beschränkt. Es können je nach Applikation bestimmte Schritte weggelassen werden, oder es können je nach Applikation weitere Schritte hinzugefügt werden.

Die vorliegende Erfindung ist nicht auf die dargestellten Ausführungsformen beschränkt, sondern es sind Änderungen ebenfalls vom Umfang der Erfindung umfasst, der durch die beigefügten Ansprüche definiert ist.

## Patentansprüche

1. Flow-Through-Chip aus einem porösen Material (1) mit einer Vielzahl durchgehender Kanäle (2), wobei der Durchmesser der Kanäle (2) zwischen 0,1 und 100 µm beträgt, so dass vorzugsweise eine einzelne eukaryontische Zelle (13) darin Platz findet,
wobei die Kanäle (2) von der Ober- und Unterseite des Flow-Through-Chips variabel, jedoch nicht unabhängig voneinander verschließbar sind durch verschiebbare Deckelplatten (3, 6) aus Polymermaterial oder Membranen; mit einer dazwischen angeordneten Dichtung (5), die bei einer angelegten elektrischen Spannung als ein Heizelement zum Erwärmen der Kanäle (2) dient, um eine Polymerase-Kettenreaktion (PCR) durchzuführen, und
eine Durchleitung von Flüssigreagenzien durch die Kanäle (2) möglich ist und
mit einem oder mehreren von:
monoklonalen Antikörpern (11) an den Innenwänden der Kanäle (2), um statistisch eine einzige gesuchte Zelle (13) pro Kanal (2) zu separieren; oder
immobilisierten Fängermolekülen (12), insbesondere Antikörpern oder einzelsträngigen Oligonukleotiden, an den Innenwänden der Kanäle (2), um gesuchte Zellen (13), gesuchte Zielmoleküle, gesuchte Zielproteine oder gesuchte Gensequenzen (15) zu binden.

2. Flow-Through-Chip gemäß Anspruch 1, wobei das poröse Material (1) als eine Nanotiterplatte oder als ein Nanotiterchip ausgebildet ist.

3. Flow-Through-Chip gemäß Anspruch 1 oder 2, wobei das poröse Material (1) aus porösem Glas oder porösem Kunststoff gebildet ist.

4. Flow-Through-Chip gemäß Anspruch 1 oder 2, wobei das poröse Material (1) aus porösem Silizium gebildet ist.

5. Flow-Through-Chip gemäß Anspruch 4, wobei das poröse Silizium (1) in Form eines Einkristallplättchens vorliegt, in das die Kanäle (2) durchgehend eingeätzt sind.

6. Verwendung eines Flow-Through-Chips aus porösem Material (1) mit einer Vielzahl durchgehender Kanäle (2), wobei der Durchmesser der Kanäle (2) zwischen 0,1 und 100 µm beträgt, und das poröse Material (1) gemäß einem der Ansprüche 1 bis 5 ausgebildet ist, zumindest zur Zellseparation, zur Zelllyse und Aufreinigung von Zielmolekülen, zur Amplifikation von Nukleinsäuremolekülen und/oder zur Detektion von gesuchten Zielmolekülen.

7. Analyseverfahren unter Verwendung eines Flow-Through-Chips aus porösem Material (1) mit einer Vielzahl Kanäle (2) mit einem Durchmesser zwischen 0,1 und 100 µm, zumindest mit:
einem Schritt zur Zellseparation in den Kanälen (2);
einem Schritt zur Zelllyse und Aufreinigung von Zielmolekülen in den Kanälen (2);
einem Schritt zur Amplifikation von Nukleinsäuremolekülen (15) in den Kanälen (2); oder
einem Schritt zur Detektion von gesuchten Zielmolekülen in den Kanälen (2).

8. Analyseverfahren gemäß Anspruch 7, wobei
bei dem Schritt zur Zellseparation ein histologischer Schnitt (8) auf die Oberfläche des porösen Materials (1) aufgebracht wird, und die Zellen (13) des histologischen Schnittes (8) vereinzelt werden, so dass statistisch eine einzige Zelle (13) pro Kanal (2) gebunden wird;
bei dem Schritt zur Zelllyse und Aufreinigung der Zielmoleküle die in den Kanälen (2) befindlichen Zellen (13) mittels immobilisierter Fängermoleküle (12), wie insbesondere Antikörper oder Oligonukleotide, innerhalb eines jeden Kanals (2) festgehalten werden und biologisch, chemisch mittels Lysereagenzien, thermisch, per Ultraschall oder mechanisch/physikalisch lysiert werden;
bei dem Schritt zur Amplifikation von Nukleinsäuremolekülen (15) die Kanäle (2) mittels eines Heizelementes erwärmt werden, um eine Polymerase-Kettenreaktion (PCR) durchzuführen; und
bei dem Schritt zur Detektion von gesuchten Zielmolekülen an den Innenwänden der Kanäle (2) immobilisierte spezifische Fängermoleküle (17), insbesondere Antikörper, Haptene oder Oligonukleotide mit den gesuchten Zielmolekülen in Interaktion treten, danach ungebundene markierte Moleküle aus den Kanälen (2) durch Hindurchpumpen von Puffer herausgewaschen werden, und danach verbleibende über die Zielmoleküle mit den Fängermolekülen (17) gebundene Marker mittels optischer, magnetische, elektrochemischer oder radioaktiver Verfahren quantifiziert werden.
